# EUROPEAN PATENT SPECIFICATION

(11) **EP 1 841 491 B1**
(45) Date of publication and mention of the grant of the patent: **27.05.2009**
(21) Application number: 05851357.3
(22) Date of filing: 31.10.2005
(51) Int. Cl.: A61M 37/00

(54) **Transdermal drug delivery device**
Transdermale Arzneimittelzuführungvorrichtung
Dispositif d'administration de medicaments transdermique

(30) Priority: 02.12.2004 US 1367
(43) Date of publication of application: 10.10.2007
(73) Proprietor: Janisys Limited, Galway (IE)
(72) Inventor: NICKEL, Janice H, Palo Alto, California 94304-1100 (US)
(74) Representative: Gorman, Francis Fergus
(86) International application number: PCT/US2005/039927
(87) International publication number: WO 2006/060106

(56) References cited:
- WO-A-01/49346
- WO-A-02/100474
- WO-A-03/035167
- WO-A-03/037403
- WO-A-03/059431
- WO-A-03/066128
- US-A- 6 132 755
- US-A1- 2002 020 688
- US-A1- 2004 176 732

## Description

### FIELD OF THE INVENTION

The present invention relates to a transdermal drug delivery device.

### BACKGROUND

Various techniques are known for delivering drugs into humans and animals. A more common set of these techniques include orally delivered drugs, such as pills or capsules, transdermally delivered drugs, such as, syringes or catheters, and transdermal patches. While typically effective for drug delivery, these techniques have certain drawbacks. For instance, the effectiveness of orally delivered drugs is often reduced due to degradation caused in the digestive system. The use of syringes or catheters typically require administration by a person trained in their use and are often associated with pain and local damage to the skin. Transdermal patches often have limited applicability due to the inability of larger molecules to penetrate the dermal layer.

Another, more recently developed technique includes the use of patches having micromachined needles formed in an array. These patches are typically fabricated to include a very large number of microneedles configured to penetrate across the dermal barrier. Although these patches have been found to be effective in enabling relatively painless drug delivery, they do have some shortfalls. For instance, the drugs contained in these patches are delivered at the time that these patches are applied onto a user's skin. More particularly, these patches are often designed such that the drugs are released into the user's skin through application of force during placement of these devices. As such, the user is typically required to apply a number of different types of these patches at different times during each day to receive prescribed amounts of the drugs contained in the patches. This may prove difficult for certain people as they may forget to administer certain ones of the drugs.

U.S. Patent Specification No. 6,132,755 of Eicher et al discloses a transcorneal drug delivery device which comprises a housing which defines a compartment, within which a reservoir for a drug is formed. A plurality of microneedles extend from the housing and communicate with the reservoir. A lid closes the comportment of the housing, and an energy supply in the form of a battery is mounted on the lid. Electronic controls are also mounted on the lid. A piezoelectric membrane located in a pumping chamber pumps the drug from the reservoir through the microneedles.

PCT Published Application Specification No. WO 01/49346 of Ackley discloses a drug delivery device which comprises a plurality of arrays of microneedles extending from corresponding housings, each of which housings forms a reservoir for a drug. The housings and microneedles are stacked so that the microneedles from one housing extend into the microneedles of the next adjacent housing for delivering a mixture of drugs from the reservoirs of the respective housings.

PCT Published Application Specification No. WO 03/035167 of Mavor et al discloses a device for controlled delivery of a drug into the skin. The device comprises a dermal patch type device within which is located a thin flexible electrochemical battery cell with respective positive and negative electrodes. A separator unit is located between the electrodes and acts to contain a pharmacologically or physiologically acceptable formulation for cosmetic or therapeutic use to be applied to the skin. The pharmaceutically acceptable or physiologically acceptable formulation is electrically conductive for facilitating iontophoretic application of the formulation to the skin of a subject.

Accordingly, it would be beneficial to have a more flexible drug delivery device capable of delivering a relatively wide variety of drugs on a prescribed delivery schedule.

### SUMMARY

A transdermal drug delivery device is described herein. The transdermal drug delivery device includes a cassette and a lid that is attachable to the cassette. The cassette includes a first reservoir for containing a drug and microneedles for delivering the drug. At least one of the microneedles is in fluid communication with the first reservoir. The lid includes a power source and an electronic device configured to receive electrical energy generated from the power source. The drug delivery device also includes a logic device configured to selectively control delivery of the electrical energy to the electronic device, whereby delivery of the electrical energy causes the electronic device to deliver the drug contained in the first reservoir. The power source comprises an electrolye material in a second reservoir formed in the cassette and electrodes located such that the power source becomes active when the lid is placed on the cassette.

### BRIEF DESCRIPTION OF THE DRAWINGS

Features of the present invention will become apparent to those skilled in the art from the following description with reference to the figures, in which:
FIG. 1A shows a simplified cross-sectional side view of a transdermal drug delivery device according to an embodiment of the invention;
FIG. 1B shows a simplified cross-sectional side view of a transdermal drug delivery device according to a second embodiment of the invention;
FIG. 1C illustrates a simplified plan view of a cassette of the transdermal drug delivery device illustrated in FIG. 1B;
FIG. 1D illustrates simplified bottom view of a lid of the transdermal drug delivery device illustrated in FIG. 1B;
FIG. 2 illustrates a block diagram of a control system for controlling a transdermal drug delivery device, such as, the transdermal drug delivery device depicted in FIGS. 1A-1D, according to an embodiment of the invention;
FIGS. 3A and 3B, illustrate simplified schematic illustrations, in cross-section, of delivery mechanisms according to two embodiments of the invention;
FIG. 4 illustrates a flow diagram of an operational mode for delivering at least one drug with a transdermal drug delivery device, according to an embodiment of the invention; and
FIG. 5 illustrates a computer system, which may be employed to perform various functions described herein, according to an embodiment of the invention.

### DETAILED DESCRIPTION

For simplicity and illustrative purposes, the present invention is described by referring mainly to an exemplary embodiment thereof. In the following description, numerous specific details are set forth in order to provide a thorough understanding of the present invention. It will be apparent however, to one of ordinary skill in the art, that the present invention may be practiced without limitation to these specific details. In other instances, well known methods and structures have not been described in detail so as not to unnecessarily obscure the present invention.

As described in greater detail herein below, a transdermal drug delivery device includes a power source to supply power and/or current to one or more components of the delivery device. A logic device configured to, for instance, determine when a drug contained in the delivery device is scheduled to be released may control the power source. In addition, the logic device may also control electrical devices, for instance, delivery mechanisms, actuators, switches, multiplexing structures, etc., configured to cause the drug to be released. By way of example, the logic device may receive input from one or more input sources, for instance, timers, sensors, etc., and may provide output to the electrical devices. In addition, the logic device may control delivery of the electrical energy from the power source to the electrical devices.

The transdermal drug delivery device also includes a cassette configured with reservoirs. The reservoirs may individually hold one or more types of drugs, such that, the drugs contained in one of the reservoirs may be kept separate from the drugs contained in other reservoirs. In addition, the reservoirs may be in fluid communication with an array of microneedles, through which the one or more types of drugs may be released from the reservoirs. The microneedles may have lengths of between about 1 µm to 1 mm. More particularly, the microneedles may be sized and configured to deliver drugs contained in the reservoirs to a user through a dermal layer of the user's skin. In addition, a diffusion barrier material may be positioned at an interface between the reservoirs and the microneedles to substantially prevent loss of the drugs until the drugs are deliberately released.

The drug delivery device further includes a lid configured to perform a number of functions in the drug delivery device. In one respect, the lid is configured to cover the reservoirs of the cassette to thereby seal the individual reservoirs. The lid may thus, for instance, include seals to substantially prevent leakage of the drugs from the reservoirs and the mixing of drugs is different reservoirs. In another respect, the lid may house the logic device, the power source, and the electrical devices. In addition, the lid may contain conductive pathways for conveying signals and power between the logic device, the power source, and the electrical devices.

The lid may be removably attached to the cassette such that the reservoirs may be easily accessed. In this regard, the materials, for instance, drugs, electrolytes, or other materials, contained in the reservoirs may be added or removed with the lid removed, In one example, the materials may be deposited into their respective reservoirs through any reasonably suitable known manner.

As stated herein above, the electrical devices may include delivery mechanisms. Generally defined, the delivery mechanisms comprise devices or actuators configured to cause the drugs contained in the reservoirs to be released through the microneedles when the delivery mechanisms are activated by the logic device. By way of example, the delivery mechanisms operate to displace the drugs contained in the reservoirs by applying force on the drugs and causing the drugs to be expelled.

If a diffusion barrier is used to prevent premature delivery of the drugs through the microneedles, the deliver mechanisms may include means for rupturing or otherwise deactivating the diffusion barrier. For instance, if the diffusion barrier is a thin membrane, the delivery mechanism may apply sufficient force to rupture the thin membrane. If the barrier is an environmentally sensitive hydrogel, the delivery mechanism may provide environmental stimuli to shrink the hydrogel to permit the release of the drugs. In this example, the hydrogel may comprise a negatively thermosensitive hydrogel and the delivery mechanism may be configured to apply heat to the hydrogel to thereby cause the hydrogel to shrink. Following shrinkage of the hydrogel, the hydrogel may be expelled from the reservoir and the drugs may be relatively freely expelled from the reservoir. Otherwise, the drugs may pass around the hydrogel to be expelled from the reservoir.

As another example, the delivery mechanism may comprise a heater configured to vaporize a liquid, the vaporization of which causes the drugs to be expelled through the microneedles. The force created through the vaporization of the liquid may be sufficient to rupture a thin membrane positioned at the interfaces between the reservoirs and the microneedles. The liquid in this example may be contained in an elastic membrane or an elastic barrier layer may be positioned between the liquid and the drugs to substantially prevent the liquid and the drugs from mixing.

As a further example, the delivery mechanism may comprise an apparatus configured to enable the initiation of a chemical reaction which creates sufficient force to cause the drugs to be expelled from the reservoirs. For example, the delivery mechanism may comprise an activation mechanism that allows the combination of various chemicals. The chemicals may include, for instance, baking soda and acetic acid, the combination of which produces carbon dioxide. The force created through the chemical reaction may be sufficient to rupture a thin membrane positioned at the interfaces between the reservoirs and the microneedles. In addition, the chemical reaction may occur in an elastic membrane or an elastic barrier layer may be positioned between the chemicals and the drugs to substantially prevent the chemicals and the drugs from mixing.

In any regard, the power source may comprise any reasonably suitable form capable of providing sufficient power and/or current to operate the sensors and the electrical devices of the delivery device. The device has an on-board battery created from a number of reservoirs containing electrolytes for providing electrical energy to a number of electrical devices configured on the delivery device. Terminals or electrodes are provided around the electrolytes to form the power source for the electrical devices. In one respect, the power source may become active when the electrodes are contacted with one another, which may occur as the lid is placed on the cassette.

Through implementation of the various examples described herein, the timing at which a drug is delivered from a microneedle equipped cassette may be controlled such that prescribed amounts of the drug may be administered to a user at various times during one or more days. In addition, a plurality of different types of drugs may be delivered to the user at the various times. In one regard, a user therefore could receive all of the medication they require for the specified time period through application of the transdermal drug delivery device described herein. Moreover, the drug delivery device may maintain one or more drugs in a pharmakinetic therapeutic region by delivering relatively small doses at relatively shorter time intervals. The drug delivery device may also be employed to accurately time the delivery of the one or more drugs to substantially prevent adverse reactions to certain mixing of drugs, to substantially prevent accidental over or under dose levels, etc.

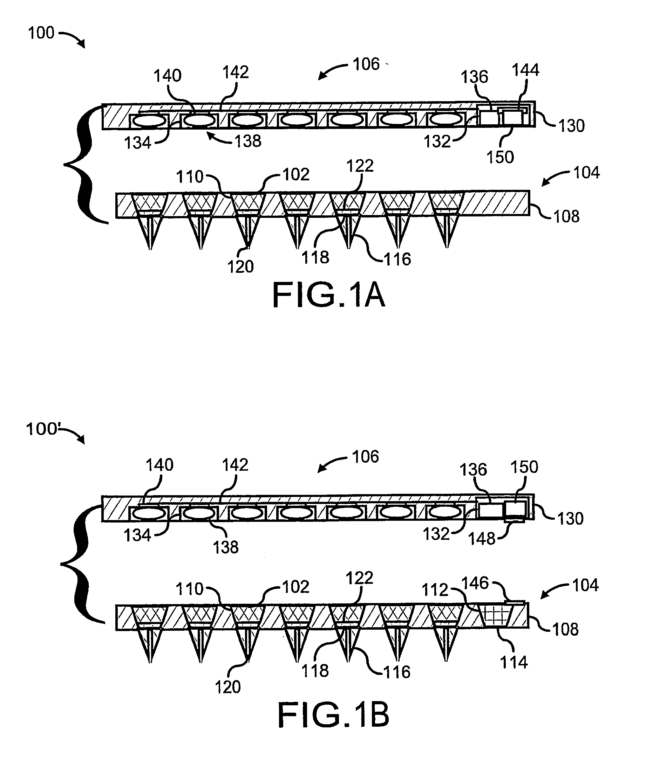

With reference to FIG. 1A, there is shown a simplified cross-sectional side view of a transdermal drug delivery device 100. It should be readily apparent that the transdermal drug delivery device 100 depicted in FIG. 1A represents a generalized illustration and that other elements may be added or existing elements may be removed or modified without departing from a scope of the transdermal drug delivery device 100. For example, the transdermal drug delivery device 100 may include additional layers, additional reservoirs and microneedles, etc.

The transdermal drug delivery device 100 is generally configured to receive and store a drug 102, which may include various known or heretofore known medicines or other agents. The drug 102 may also include medicines that are known to be administered either transdermally or through other means, such as, orally, subcutaneously, pulmonarily, etc. The transdermal drug delivery device 100 is also configured to be placed on a user's skin such that the drug 102 contained in the delivery device 100 may be delivered transdermally. In this regard, the transdermal drug delivery device 100 may optionally be equipped with adhesives or the like to enable the device 100 to remain adhered to the user's skin for a period of time. As described in greater detail hereinbelow, the transdermal drug delivery device 100 is equipped with mechanisms designed to control the release of the drug 102 into the user's skin at prescribed times.

The transdermal drug delivery device 100 is illustrated in FIG. 1A as including a cassette 104 and a lid 106. The cassette 104 includes a substrate 108 having a plurality of reservoirs 110, 112 formed throughout the substrate 108. The substrate 108 may be constructed from any reasonably suitable material. Suitable materials may include, for instance, silicon, metals, ceramics, polymers, composites and the like. In addition, the substrate 108 may be formed of flexible or rigid materials.

A plurality of microneedles 116 are formed on a lower surface of the substrate 108. The microneedles 116 are formed such that they are in fluid communication with one or more of the reservoirs 110 through respective openings 118. As shown in FIG. 1A, however, the microneedles 116 are each in fluid communication with a respective one of the reservoirs 110. In any respect, the microneedles 116 are sized and shaped to penetrate the stratum corneum layer of a user's skin. In addition, the microneedles 116 include channels 120 having sufficient diameters to permit passage of the drug 102 contained in the reservoirs 110 through the microneedles 116. In one example, the microneedles 116 may have lengths ranging from about 1 µm to 1 mm and the substrate 108 may include an array of 100 or more microneedles 116.

The openings 118 at the interfaces between the reservoirs 110 and the microneedles 116 may be covered with respective membranes 122. Examples of suitable materials for the membranes 122 comprise polymers, ceramics, metals, glasses, hydrogels, etc. The membranes 122 are configured to provide a liquid seal of the reservoirs 110 and to substantially prevent contamination of the drugs 102 contained in the reservoirs 110. The membranes 122 are also configured to rupture or otherwise enable the drugs 102 contained in the reservoirs 110 to flow through the openings 118 when desired. In one example, the membranes 122 are configured to rupture when at least a predetermined amount of force is exerted on the membranes 122. In this regard, the timing of exertion of pressure on the membranes 122 may be controlled to thus control the release of the drugs 102, as described in greater detail herein below.

The cassette 104 and the lid 106 may be formed through any number of reasonably suitable manufacturing techniques. For instance, the cassette 104, including the reservoirs 110, 112 and the microneedles 116, may be formed using standard MEMS (MicroElectro-Mechanical System) manufacturing techniques. In addition, the cassette 104 and the lid 106 may be formed using other methods known to those skilled in the art.

The lid 106 may be attached to the cassette 104 to provide a liquid seal of the drugs 102 contained in the reservoirs 110. In this regard, the lid 106 may be bonded to the cassette 104 through use of an adhesive (not shown). The adhesive may, for instance, be pressure-activated, heat-activated, or the like. In addition, the adhesive may be selected to provide an adequate seal at the interface between the lid 106 and the cassette 104, such that, any drug 102 that may have been released from the reservoirs 110 may substantially be prevented from leaking out of the transdermal drug delivery device 100. The lid 106 may also substantially prevent the mixing of drugs 102 contained in different reservoirs 110.

As an alternative to the use of adhesives, the lid 106 may be attached to the cassette 104 through other suitable means. For instance, the lid 106 or the cassette 104 may be formed of a material designed to be bonded to the cassette 104 through application of heat, light, or other types of energy. As another example, the lid 106 and the cassette 104 may be formed with complimentary structures configured to mate with one another and provide an interlocking connection between the lid 106 and the cassette 104.

In any respect, the lid 106 may be attached to the cassette 104 following insertion of the drugs 102 into the reservoirs 110. In addition, although the lid 106 is shown as being separate from the cassette 104, the lid 106 may be integrally formed with the cassette 104. In this instance, the lid 106 may be attached to the cassette 104 through use of a hinge (not shown) which enables access to the reservoirs 110.

As shown in FIG. 1A, the lid 106 includes a substrate 130 having a plurality of cavities 132, 134 formed in the substrate 130. The cavities 132, 134 may be formed through any reasonably suitable manner known to those skilled in the art. For instance, the cavities 132, 134 may be formed through MEMS fabrication techniques, etching, lithography, etc. In any regard, the cavities 132, 134 house various components of the transdermal drug delivery device 100.

In the example shown in FIG. 1A, the first cavity 132 houses a logic device 136 and an input source 150. Examples of suitable input sources 150 include, for instance, clocks, timers, sensors, switches, etc. The input source 150 generally operates as an input source for the logic device 136. More particularly, the logic device 136 may employ the information received from the input source 150 in controlling operations of various electronic devices contained in or on the delivery device 100. Although the logic device 136 and the input source 150 have been illustrated as being located within the first cavity 132, it should be understood that the logic device 136 and the input source 150 may be positioned externally to the lid 106 without deviating from a scope of the delivery device 100 described herein.

The electronic devices may include, for instance, delivery mechanisms 138, which are illustrated in FIG. 1A as being housed in the second cavities 134. Again, it should be understood that part or all of the delivery mechanisms 138 may be positioned externally to the lid 106 without departing from a scope of the delivery device 100 described herein. The positioning of the delivery mechanisms 138 may be based upon the configurations of the delivery mechanisms 138.

In any respect, the delivery mechanisms 138 generally operate to enable delivery of the drugs 102 and may comprise various configurations as described in greater detail herein below. As shown, the delivery mechanisms 138 each include an actuating mechanism 140 configured to receive electrical energy through conductive pathways 142 formed or contained in the substrate 130. The electrical energy may be supplied into the conductive pathways 142 from a power source 144. The power source 144 may comprise any reasonably suitable power source that may be housed in delivery device 100. Thus, the power source 144 illustrated in FIG. 1A is for purposes of illustration and is not intended to limit the delivery device 100 in any respect. In this respect, the power source 144 may be located, for instance, at any position in or on the lid 106.

In general, the power source 144 may comprise any reasonably suitable form capable of providing sufficient power and/or current to operate the sensors and the electrical devices of the delivery device 100. An example of a suitable power source 144 may include a thin film battery incorporated into or positioned on the lid 106 of the delivery device 100. Another example, which is shown in FIGS. 1B-1D, is an on-board battery created from a number of reservoirs 112 containing electrolytes 114 for providing the electrical energy.

With particular reference to FIG. 1B, there is shown a simplified cross-sectional side view of a transdermal drug delivery device 100' according to a second example. Initially, it should be understood that elements in FIG. 1B having like reference numerals as those depicted in FIG. 1A correspond to the same elements in FIG. 1A and vice versa. Therefore, a detailed description of those like elements are omitted as having already been described with respect to FIG. 1A.

As shown, the substrate 108 of the cassette 104 includes reservoirs 112 containing electrolytes 114. In addition, terminals or electrodes 146, 148 are provided around the electrolytes 114 to form a power source for the delivery device 100. In one respect, the power source may become active when the electrodes 146, 148 are contacted with one another, which may occur as the lid is placed on the cassette. In addition, the electrical energy generated from the electrolytes 114 and the electrodes 146, 148 may be delivered to various electronic devices, for instance, the delivery mechanisms 138 through the conductive pathways 142.

The electrolytes 114 and the electrodes 144, 146 may comprise any reasonably suitable materials generally known to be used by those skilled in the art to generate electrical energy. In this regard, a detailed description of the general mechanics behind the generation of electrical energy through use of electrolytes and electrodes is omitted.

As additionally shown, the reservoirs 112 have been illustrated without respective microneedles 116 because the electrolytes 114 are not intended to be ejected from the cassette 104. However, if microneedles 116 are formed beneath the reservoirs 112, openings between the reservoirs 112 and the microneedles 116 may be capped to prevent leakage of the electrolytes 114.

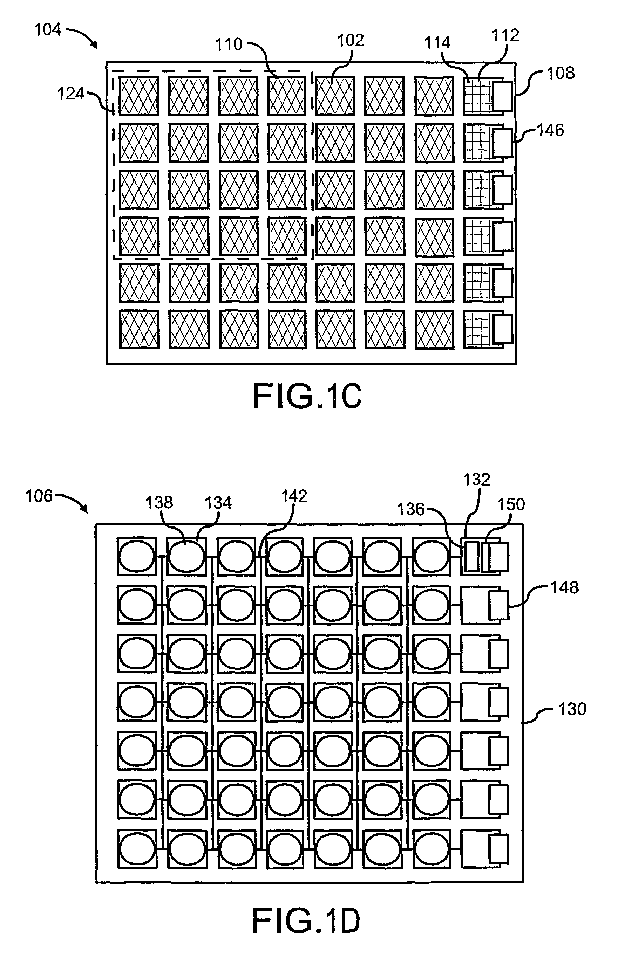

Turning now to FIG. 1C, there is shown a simplified plan view of the cassette 104 illustrated in FIG. 1B. It should be readily apparent that the cassette 104 illustrated in FIG. 1C represents a generalized illustration and that other elements may be added or existing elements may be removed or modified without departing from a scope of the cassette 104. It should also be understood that the number of reservoirs 110,112 depicted in FIG. 1C is not meant to limit the cassette 104 in any respect but have been so illustrated to provide a thorough understanding of a cassette 104 according to one example.

As shown in FIG. 1C, a number of reservoirs 110, 112 are positioned in an array on the cassette 104, such that, the cassette 104 may include a relatively large number of reservoirs 110, 112. The reservoirs 110 may hold different types of drugs 102. For instance, the reservoirs 110 contained in the outlined section 124 may be configured to hold a first type of drug 102, whereas the reservoirs 110 located outside of the outlined section 124 may hold a second type of drug 102. In addition, the reservoirs 110 may hold any reasonably suitable number of drugs 102 in any reasonably suitable arrangement. In this regard, a single cassette 104 may be used to transdermally deliver any reasonably suitable number of drugs 102 to a user. In addition, the times or frequencies at which the various drugs 102 are delivered to a user may also be controlled. Thus, a user who is required to receive various medications at various times during a day, for instance, may do so through use of a single cassette 104.

The electrolytes 114 are also shown as being arranged in separately formed reservoirs 112. It should be understood that the number of reservoirs 112 containing the electrolytes 114 is for purposes of illustration and is not meant to limit the transdermal drug delivery device 100 in any respect. Instead, any reasonably suitable number of reservoirs 112 may be employed to contain the electrolytes 114. In addition, the number of reservoirs 112 containing the electrolytes 114 may be selected, for instance, according to the amount of electrical energy required to operate the delivery device 100.

Referring now to FIG. 1D, there is shown a simplified bottom view of the lid 106 illustrated in FIG. 1B. It should be readily apparent that the lid 106 illustrated in FIG. 1D represents a generalized illustration and that other elements may be added or existing elements may be removed or modified without departing from a scope of the delivery device 100 described herein. It should also be understood that the number of components depicted in FIG. 1D is not meant to limit the lid 106 in any respect but have been so illustrated to provide a thorough understanding of a lid 106 according to one example.

As shown in FIG. 1D, a number of cavities 132, 134 are positioned in an array on the lid 106, such that, the cavities 132, 134 substantially align with respective ones of the reservoirs 110, 112 in the cassette 104. In addition, the electrodes 148 positioned on the lid 106 are configured to contact respective ones of the electrodes 146 positioned on the cassette 104. In this regard, when the lid 106 is positioned on top of the cassette 104, the electrodes 146, 148 and the electrolytes 114 are configured to generate electrical energy and therefore operate as a power source for the drug delivery device 100. As described in greater detail herein below, the electrical energy may be used to power one or more electrical devices, input sources, a logic device, etc., in delivering the drugs 102 to a user.

Although a single logic device 136 and input source 150 have been illustrated in FIG. 1C., additional logic devices 136 and input sources 150 may be provided in at least one of the remaining cavities 132 without departing from a scope of the lid 106. In addition, the number of cavities 132 may be reduced to thereby create larger cavities 132, for instance, in situations where at least one of the logic device 136 and the input source 150 requires additional space. Moreover, the logic device 136, as well as other components illustrated in the lid 106, such as, the input source 150, the delivery mechanisms 138, the conductive pathways 142, etc., may be positioned externally to the lid 106.

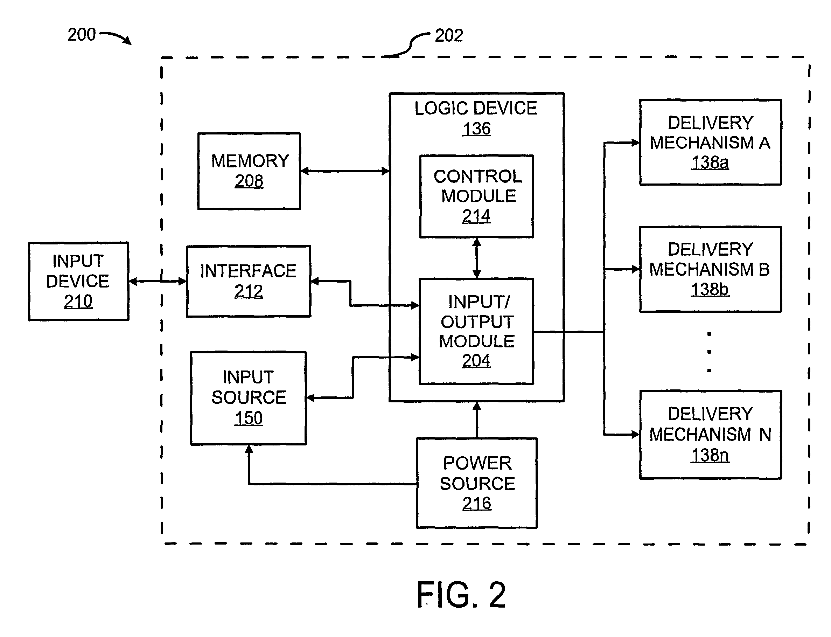

FIG. 2 depicts a block diagram 200 of a control system 202 for controlling a transdermal drug delivery device, such as, the transdermal drug delivery device 100. It should be understood that the following description of the block diagram 200 is but one manner of a variety of different manners in which such a control system 202 may be operated to control operations of a transdermal drug delivery device 100. In addition, it should be understood that the control system 202 may include additional components and that some of the components described may be removed and/or modified without departing from a scope of the control system 202. Moreover, although particular reference is made to the transdermal drug delivery device 100 depicted in FIGS. 1A-1D, it should be understood that the control system 202 may be employed to control drug delivery devices having configurations that differ from that illustrated with respect to the transdermal drug delivery device 100.

The control system 202 includes a logic device 136 configured to control various operations of the delivery device 100. The logic device 136 may, for instance, comprise a controller such as a computing device, a microprocessor, a micro-controller, an application specific integrated circuit (ASIC), and the like. In general, the logic device 136 may be programmed to receive input, to process the input, and to control when to actuate various delivery mechanisms to thereby control when one or more drugs 102 are administered to a user. The logic device 136 may be further programmed to determine whether one or more drugs 102 stored in the delivery device 100 may be likely to cause an adverse reaction with one or more other drugs 102. If the logic device 136 makes this determination, the logic device 136 may ensure that the drugs 102 are delivered at rates to substantially prevent the potential adverse reaction or to provide an indication of the potential adverse reaction.

The logic device 136 includes an input/output module 204 configured to receive instructions as well as other information from an input source 150. The input source 150 may comprise, for instance, a clock, a timer, a sensor, etc. The input/output module 204 may, in one regard, function as an adapter for the logic device 136 to receive and transmit data. In this regard, the input/output module 204 may comprise hardware and/or software configured to perform these functions. In addition, although the input/output module 204 has been illustrated as forming part of the logic device 136, the input/output module 204 may comprise an algorithm stored in a memory 208 accessible by the logic device 136. The memory 208 may also generally be configured to provide storage of software that provides the functionality of the logic device 136. The memory 208 may be implemented, for instance, as a combination of volatile and non-volatile memory, such as DRAM, MRAM, EEPROM, flash memory, and the like.

An input device 210 may be used to input instructions into the input/output module 204. The input device 210 may comprise, for instance, a user interface terminal, such as, a computing device, a handheld computer, a personal digital assistant, etc. The input device 210 may communicate with the logic device 136 through an interface 212, which may comprise hardware and/or software configured to enable information to be transferred in at least one direction from the input device 210 to the logic device 136. The communication between the input device 210 and the logic device 136 may be enabled through any reasonably suitable wired or wireless protocol.

The instructions may include, for instance, when a drug contained in the transdermal drug delivery device 100 is to be administered, how often the drug is to be administered, the quantities of the drug to be administered, which of the drugs contained in which of the reservoirs are to be administered at specific times, etc. The input device 210 may also provide instructions to the logic device 136 regarding potential adverse reactions through a combination of one or more of the drugs 102 contained in the delivery device 100. If the logic device 136 receives these instructions, the logic device 136 may ensure that the drugs 102 are delivered at rates to substantially prevent the potential adverse reaction or to provide an indication of the potential adverse reaction.

The instructions sent from the input device 210 may be similar to a prescription for person required to take one or more drugs. These instructions may be programmed into the memory 208 and may be stored as an algorithm, a look up table, etc. During operation of the transdermal drug delivery device 100, the logic device 136 may access this information in controlling various aspects of drug delivery by the transdermal drug delivery device 100.

The logic device 136 may be programmed following the supply of the at least one drug 102 into the reservoirs 110. The logic device 136 may alternatively be programmed prior to or during fabrication of the transdermal drug delivery device 100. Thus, for instance, an algorithm for controlling the logic device 136 may be pre-programmed.

The logic device 136 may include a control module 214, which may comprise hardware and/or software configured to perform various control functions of the logic device 136. Although the control module 214 has been illustrated as forming part of the logic device 136, the control module 214 may comprise an algorithm stored in the memory 208 accessible by the logic device 136. In any regard, the control module 214 may, broadly speaking, operate to receive input, process the input, and transmit control signals to act on the processed input.

In a first example, the input source 150 may comprise at least one of a clock and a timer and may transmit timing information to the control module 214. The control module 214 may process the timing information to determine whether one or more of the delivery mechanisms 138a-138n are to be activated to deliver the drugs 102 contained in one or more reservoirs 110. More particularly, for instance, the control module 214 may be programmed to deliver the drugs 102 contained in one or more of the reservoirs 110 at a particular time or after a particular amount of time has elapsed. In this regard, the control module 214 may track the passage of time determined by the input source 150 to determine when to deliver the drugs 102. In addition, the control module 214 may operate to selectively control delivery of electrical energy to particular ones of the delivery mechanisms 138a-138n in order to cause the drugs 102 associated with those delivery mechanisms 138a-138n to be delivered.

In a second example, the input source 150 may comprise a sensor configured to detect at least one condition. In one example, the input source 150 may be positioned to detect at least one condition in a user's bloodstream. For instance, the input source 150 may be positioned and configured to detect the glucose level in the blood. The input source 150 may also be positioned and configured to monitor any reasonably suitable drug or biological marker data. In this example, the control module 214 may process the detected condition information and selectively control the delivery of the drugs based upon the detected condition information. For instance, if the detected condition information indicates that the glucose level is too high, the control module 214 may determine that insulin is required to reduce the glucose level.

In another example, the input source 150 may be configured to detect one or more environmental conditions. For instance, the input source 150 may be configured to detect airborne particulates, such as, nerve agents and other potentially harmful chemicals. In this example, the control module 214 may discern the agent and may determine an appropriate antidote for the agent. In this regard, the transdermal drug delivery device 100 may store a number of different antidotes for a number of different agents.

In either of the examples above, the control module 214 may track one or more conditions as detected by the input source 150 to determine when to deliver the drugs 102. In addition, the control module 214 may operate to selectively control delivery of electrical energy to particular ones of the delivery mechanisms 138a-138n in order to cause the drugs 102 associated with those delivery mechanisms 138a-138n to be delivered.

Electrical energy may be supplied to the logic device 136, the input source 150, and various other electrical devices from a power source 216. The power source 216 may comprise any reasonably suitable form capable of providing sufficient power and/or current to operate the sensors and the electrical devices of the delivery device 100. An example of a suitable power source 216 may include a thin film battery incorporated into or positioned on the lid 106 of the delivery device 100, as shown in FIG. 1A. Another example, which is shown in FIGS. 1B-1D, is an on-board battery created from a number of reservoirs 112 containing electrolytes 114 for providing the electrical energy.

The control module 214 may control delivery of the electrical energy to various ones of the delivery mechanisms 138a-138n. In one example, each of the delivery mechanisms 138a-138n may be addressed through use of multiplexers/demultiplexers. The use of multiplexers/demultiplexers is generally known, for instance, to address particular locations on a grid through row and column designations and is thus not described in greater detail here. In any respect, however, the control module 214 may determine when the delivery mechanisms 138a-138n are to selectively receive the electrical energy to thereby cause the drugs 102 contained in associated reservoirs 110 to be released. As described above, this determination may be made based upon information received from the input source 150.

The delivery mechanisms 138a-138n may comprise various forms. Generally defined, the delivery mechanisms 138a-138n may comprise devices or actuators configured to cause the drugs 102 contained in the reservoirs 110 to be released through the microneedles when the delivery mechanisms 138a-138n are activated by the logic device 136. By way of example, the delivery mechanisms 138a-138n may operate to displace the drugs 102 contained in the reservoirs 110 by applying force on the drugs 102 and causing the drugs 102 to be expelled.

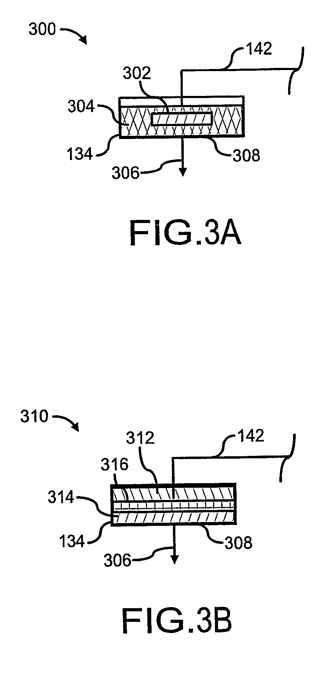

In a first example, the membrane 122 may comprise a hydrogel configured to shrink under various environmental conditions. In this example, the delivery mechanisms 138a-138n may comprise elements configured to provide the necessary environmental stimuli to shrink the hydrogel to permit the release of the drugs 102. For instance, the delivery mechanisms 138-138n may comprise heating elements 302 (FIG. 3A) configured to sufficiently increase the temperature of the hydrogel to cause the hydrogel to shrink and thereby enable the drugs to be delivered from the reservoirs 110.

In another example, the delivery mechanism 138a-138n may comprise heating elements 302 configured to vaporize a liquid 304, the vaporization of which causes the drugs 102 to be expelled through the microneedles 116. FIG. 3A depicts a simplified schematic illustration, in cross-section, of a delivery mechanism 300 comprising the heating elements 302. The heating elements 302 may generally comprise any reasonably suitable device configured to become heated to a prescribed level as electrical energy flows through the device.

In the example illustrated in FIG. 3A, when the logic device 136 determines that the delivery mechanism 300 is to become activated, the logic device 136 causes electrical energy to be supplied to the heating element 302 through the conductive pathway 142. The heat generated by the heating element 302 causes the liquid 304 to vaporize and expand in the second cavity 134. The vaporization of the liquid 304 causes expansion in the direction shown by the arrow 306. In one respect, an interface 308 between the second cavity 134 and the drug 102 may comprise an elastic material configured to deform as the liquid 304 vaporizes. Alternatively, the liquid 304 may be substantially encapsulated in an elastic material. In any regard, the force created through the vaporization of the liquid 304 may provide sufficient expansion to force the drug 102 to be expelled through the microneedle 116.

As a further example, the delivery mechanisms 138a-138n may comprise apparatuses configured to enable the initiation of a chemical reaction which creates sufficient force to cause the drugs 102 to be expelled from the reservoirs 110. FIG. 3B depicts a simplified schematic illustration, in cross-section, of a delivery mechanism 310 comprising these apparatuses. The delivery mechanism 310 is illustrated with a first chemical 312 and a second chemical 314 for purposes of simplicity and not of limitation. Thus, it should be understood that any number of chemicals may be employed without deviating from a scope of the delivery mechanism 310. Also shown in FIG. 3B is an activation mechanism 316 positioned between the first chemical 312 and the second chemical 314. The activation mechanism 316 may comprise any reasonably suitable device configured to enable combination of the first chemical 312 and the second chemical 314 through receipt of electrical energy. In addition, the first chemical 312 and the second chemical 314 may be selected from any reasonably suitable elements whose combination creates expansion and the application of sufficient force to cause the drug 102 to be expelled from the reservoir 110. Examples of suitable chemicals 312 and 314 include, for instance, baking soda and acetic acid, the combination of which produces carbon dioxide.

In the example illustrated in FIG. 3B, when the logic device 136 determines that the delivery mechanism 310 is to become activated, the logic device 136 causes electrical energy to be supplied to the activation mechanism 316 through the conductive pathway 142. The receipt of electrical energy by the activation mechanism 316 causes a barrier between the first chemical 312 and the second chemical 314 to be removed, thereby enabling the first chemical 312 and the second chemical 314 to mix. The mixing of the first chemical 312 and the second chemical 314 causes expansion in the direction shown by the arrow 306. In one respect, an interface 308 between the second cavity 134 and the drug 102 may comprise an elastic material configured to deform as the mixture expands. Alternatively, the mixture may be substantially encapsulated in an elastic material. In any regard, the force created through the chemical reaction between the first chemical 312 and the second chemical 314 may provide sufficient expansion to force the drug 102 to be expelled through the microneedle 116.

Various manners in which the control system 202 may be employed to deliver at least one drug 102 to a user from a transdermal drug delivery device 100 will now be described in greater detail with respect to the following flow diagram.

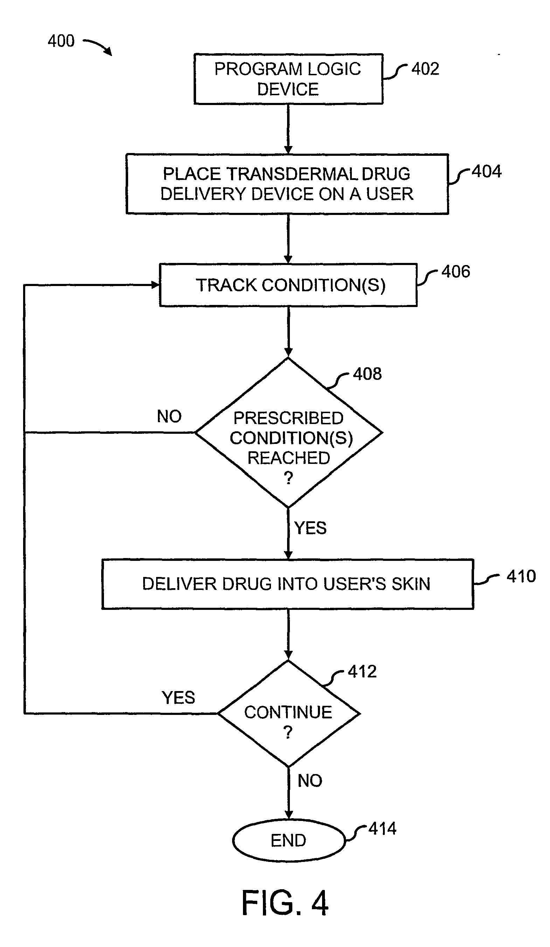

With reference to FIG. 4, there is shown a flow diagram of an operational mode 400 for delivering at least one drug 102 with a transdermal drug delivery device 100. It is to be understood that the following description of the operational mode 400 is but one manner of a variety of different manners in which the at least one drug 102 may be delivered with a transdermal drug delivery device 100. It should also be apparent to those of ordinary skill in the art that the operational mode 400 represents a generalized illustration and that other steps may be added or existing steps may be removed or modified without departing from a scope of the operational mode 400. The description of the operational mode 400 is made with reference to the block diagram 200 illustrated in FIG. 2, and thus makes reference to the elements cited therein.

Prior to initiation of the operational mode 400, the reservoirs 110 of the transdermal drug delivery device 100 may be filled with one or more drugs 102. The reservoirs 110 may be filled through use of any reasonably suitable device capable of filling the reservoirs 110 with the one or more drugs 102.

As shown in FIG. 4, a logic device 136 may be programmed at step 402. In general, the logic device 136 may be programmed to control when to actuate various delivery mechanisms 138a-138n to thereby control when drugs 102 contained in various reservoirs 110 are administered to a user. In this regard, the logic device 136 may be programmed to activate a first delivery mechanism 138a at a first time and to actuate a second delivery mechanism 138b at a second time. In addition, the logic device 136 may be programmed at any time before, during or after placement of the transdermal drug delivery device 100 on a user's skin at step 404.

At step 406, at least one condition may be tracked by the logic device 136 from information received from the input source 150. The at least one condition may comprise, for instance, timing information from a clock or a timer. In addition, or alternatively, the at least one condition may comprise a condition detected by a sensor. In the event that the input source 150 comprises a timing device, the logic device 136 may compare the timing information received with a prescribed time to determine whether the prescribed time has been reached at step 408. If the prescribed time has not been reached, the logic device 136 may continue to track the lapse oftime at step 406 until the prescribed time has been reached at step 408. If the prescribed time has been reached at step 408, the logic device 136 may cause electrical energy to be delivered to one or more of the delivery mechanisms 138a-138n to deliver the drugs 102 contained in at least one of the reservoirs 110 into the user's skin at step 410.

In the event the input source 150 comprises a sensor, the logic device 136 may compare the sensed condition with a prescribed condition to determine whether the prescribed condition has been reached at step 408. If the prescribed time has not been reached, the logic device 136 may continue to track the sensed condition at step 406 until the prescribed condition has been reached at step 408. If the prescribed condition has been reached at step 408, the logic device 136 may cause electrical energy to be delivered to one or more of the delivery mechanisms 138a-138n to deliver the drugs 102 contained in at least one of the reservoirs 110 at step 410.

In any event, at step 412, it may be determined as to whether the operational mode 400 is to continue. The operational mode 400 may be continued, for instance, if additional doses of the drug 102 or additional drugs 102 are to be delivered to the user. If it is determined that the operational mode 400 is to continue, the at least one condition may be tracked again at step 406 and steps 408-412 may be repeated substantially continuously until it is determined that the operational mode 400 is to discontinue. In this instance, the operational mode 400 may end, as indicated at step 414 and the transdermal drug delivery device 100 may be removed from the user's skin.

Through implementation of the operational mode 400, a transdermal drug delivery device 100 may be used to administer one or more drugs to a user at various prescribed times. In this regard, for instance, the user may apply a single transdermal drug delivery device 100 and may receive prescribed amounts of the one or more drugs at the prescribed times. Therefore, the user need only remember to apply the transdermal drug delivery device 100 and need not be burdened with having to remember to take the one or more drugs at the various prescribed times.

Some or all of the operations illustrated in the operational mode 400 maybe contained as a utility, program, or a subprogram, in any desired computer accessible medium. In addition, the operational mode 400 may be embodied by a computer program, which can exist in a variety of forms both active and inactive. For example, they can exist as software program(s) comprised of program instructions in source code, object code, executable code or other formats. Any of the above can be embodied on a computer readable medium, which include storage devices and signals, in compressed or uncompressed form.

Exemplary computer readable storage devices include conventional computer system RAM, ROM, EPROM, EEPROM, and magnetic or optical disks or tapes. Exemplary computer readable signals, whether modulated using a carrier or not, are signals that a computer system hosting or running the computer program can be configured to access, including signals downloaded through the Internet or other networks. Concrete examples of the foregoing include distribution of the programs on a CD ROM or via Internet download. In a sense, the Internet itself, as an abstract entity, is a computer readable medium. The same is true of computer networks in general. It is therefore to be understood that any electronic device capable of executing the above-described functions may perform those functions enumerated above.

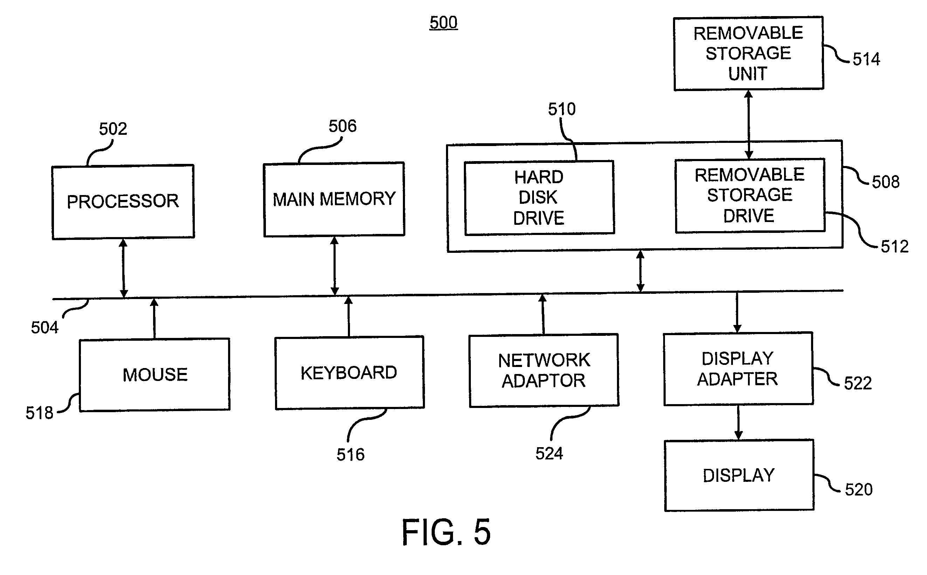

FIG. 5 illustrates a computer system 500, which may be employed to perform various functions described herein. The computer system 500 may include, for example, the controller input device 210 and/or the logic device 136. In this respect, the computer system 500 may be used as a platform for executing one or more of the functions described herein above with respect to the various components of the control system 202.

The computer system 500 includes one or more controllers, such as a processor 502. The processor 502 may be used to execute some or all of the steps described in the operational mode 400. Commands and data from the processor 502 are communicated over a communication bus 504. The computer system 500 also includes a main memory 506, such as a random access memory (RAM), where the program code for, for instance, the logic device 136 and/or the input device 210, may be executed during runtime, and a secondary memory 508. The secondary memory 508 includes, for example, one or more hard disk drives 510 and/or a removable storage drive 512, representing a floppy diskette drive, a magnetic tape drive, a compact disk drive, etc., where a copy of the program code for the control system 202 may be stored.

The removable storage drive 510 reads from and/or writes to a removable storage unit 514 in a well-known manner. User input and output devices may include a keyboard 516, a mouse 518, and a display 520. A display adaptor 522 may interface with the communication bus 504 and the display 520 and may receive display data from the processor 502 and convert the display data into display commands for the display 520. In addition, the processor 502 may communicate over a network, for instance, the Internet, LAN, etc., through a network adaptor 524.

It will be apparent to one of ordinary skill in the art that other known electronic components may be added or substituted in the computer system 500. In addiction, the computer system 500 may include a system board or blade used in a rack in a data center, a conventional "white box" server or computing device, etc. Also, one or more of the components in FIG. 5 may be optional (for instance, user input devices, secondary memory, etc.).

What has been described and illustrated herein is a preferred embodiment of the invention along with some ofits variations.

## Claims

1. A transdermal drug delivery device (100,100') comprising a cassette (104), the cassette (104) comprising a first reservoir (110) for containing a drug (102), and a plurality of microneedles (116), at least one of the microneedles (116) being in fluid communication with the first reservoir (110), a lid (106) attachable to the cassette (104) for covering the first reservoir (110), a power source (114,144), an electronic device (138) provided on the lid (106) configured to receive electrical energy generated from the power source (114,144), and a logic device (136,214) configured to selectively control delivery of the electrical energy to the electronic device (138), wherein delivery of the electrical energy causes the electronic device (138) to deliver the drug (102) contained in the first reservoir (110), **characterised in that** the power source (144) comprises an electrolyte material (114) in a second reservoir (112) formed in the cassette (104) and electrodes (146,148) located such that the power source (144) becomes active when the lid (106) is placed on the cassette (104).

2. A transdermal drug delivery device as claimed in Claim 1 **characterised in that** a first one (146) of the electrodes is positioned on the cassette (104), and a second one (148) of the electrodes is positioned on the lid (106), the first and second electrodes (146,148) being positioned with respect to the electrolyte material (114) to enable electrical energy generation when the lid (106) is placed on the cassette (104).

3. A transdermal drug delivery device as claimed in Claim 1 or 2 **characterised in that** the electronic device (138) comprises a delivery mechanism (138) configured to apply pressure on the drug (102) in response to receipt of electrical energy, said application of pressure being sufficient to cause the drug (102) to be expelled from the first reservoir (110).

4. A transdermal drug delivery device as claimed in Claim 3 **characterised in that** a diffusion barrier (122) is positioned at an interface (118) between the first reservoir (110) and the at least one microneedle (116) which is in fluid communication with the first reservoir (110), and the delivery mechanism (138) comprises one of a means (138) for rupturing the diffusion barrier (122) and a means (138) for deactivating the diffusion barrier (122) to thereby enable the drug (102) to flow out of the first reservoir (110).

5. A transdermal drug delivery device as claimed in Claim 3 **characterised in that** a hydrogel material (122) is positioned at an interface (118) between the first reservoir (110) and the at least one of the microneedle (116) which is in fluid communication with the first reservoir (110), and the delivery mechanism (138) is configured to shrink the hydrogel material (122) to thereby enable the drug (102) to be delivered from the first reservoir (110).

6. A transdermal drug delivery device as claimed in any of Claims 3 to 5 **characterised in that** the delivery mechanism (138) comprises a heating element (302) configured to vaporise a liquid (304), whereby vaporisation of the liquid (304) causes the drug (102) to be expelled from the first reservoir (110) through the at least one microneedle (116) which is in fluid communication with the first reservoir (110).

7. A transdermal drug delivery device as claimed in any of Claims 3 to 5 **characterised in that** the delivery mechanism (138) comprises an apparatus (310) configured to enable the initiation of a chemical reaction between two or more chemicals (312,314), whereby the chemical reaction creates sufficient force to cause the drug (102) to be expelled from the first reservoir (110).

8. A transdermal drug delivery device as claimed in any preceding claim **characterised in that** an input source (150) configured to supply the logic device (136,214) with at least one of timing and condition information is provided.

9. A transdermal drug delivery device as claimed in Claim 8 **characterised in that** the input source (150) comprises at least one of a clock and a timer, and the logic device (136,214) is configured to process timing information from the at least one of the clock and the timer and to deliver the electrical energy to the electronic device (138) at a prescribed time.

10. A transdermal drug delivery device as claimed in Claim 8 or 9 **characterised in that** the input source (150) comprises a sensor configured to detect at least one condition, and the logic device (136,214) is configured to process the detected condition information from the sensor, and to selectively control the delivery of the drug (102) from the reservoir (110) based upon the detected condition information.

11. A transdermal drug delivery device as claimed in any preceding claim **characterised in that** the device further comprises a plurality of first reservoirs (110), each of said plurality of first reservoirs (110) being in fluid communication with a corresponding one of the microneedles (116), and a plurality of electronic devices (13 8) associated with respective ones of said plurality of first reservoirs(110), and the logic device (136) is configured to deliver electrical energy to selected ones of the plurality of electronic devices (138) to deliver drugs contained in the associated first reservoirs (110) of the selected electronic devices (138).

## Patentansprüche

1. Transdermale Arzneimittelzuführungsvorrichtung (100, 100'), umfassend eine Kassette (104), wobei die Kassette (104) Folgendes umfasst: ein erstes Reservoir (110) für die Aufnahme eines Arzneimittels (102) und einer Vielzahl an Mikronadeln (116), wobei mindestens eine der Mikronadeln (116) in Fluidverbindung mit dem ersten Reservoir (110) steht, einen Deckel (106), welcher an der Kassette (104) angebracht werden kann, um das erste Reservoir (110) abzudecken, eine Stromquelle (114, 144), eine auf dem Deckel (106) vorgesehene elektronische Vorrichtung (138), die für die Aufnahme von durch die Energiequelle (114, 144) erzeugter elektrischer Energie konfiguriert ist, und eine Logikvorrichtung (136, 214), die für die selektive Steuerung der Zuführung der elektrischen Energie an die elektronische Vorrichtung (138) konfiguriert ist, wobei die Zuführung der elektrischen Energie bewirkt, dass die elektronische Vorrichtung (138) den in dem ersten Reservoir (110) enthaltenen Arzneistoff (102) zuführt, **dadurch gekennzeichnet, dass** die Energiequelle (144) ein Elektrolytmaterial (114) in einem in der Kassette (104) gebildeten zweiten Reservoir (112) und Elektroden (146, 148), die so angeordnet sind, dass die Energiequelle (144) aktiv wird, wenn der Deckel (106) auf die Kassette (104) gelegt wird, umfasst.

2. Transdermale Arzneimittelzuführungsvorrichtung gemäß Anspruch 1, **dadurch gekennzeichnet, dass** eine erste (146) der Elektroden auf der Kassette (104) positioniert ist und eine zweite (148) der Elektroden auf dem Deckel (106) positioniert ist, wobei die erste und zweite Elektrode (146, 148) hinsichtlich des Elekrolytmaterials (114) so positioniert ist, um die Erzeugung von elektrischer Energie zu ermöglichen, wenn der Deckel (106) auf die Kassette (104) gelegt wird.

3. Transdermale Arzneimittelzuführungsvorrichtung gemäß Anspruch 1 oder 2, **dadurch gekennzeichnet, dass** die elektronische Vorrichtung (138) einen Zuführungsmechanismus (138) umfasst, der so konfiguriert ist, um Druck auf das Arzneimittel (102) als Reaktion auf den Empfang von elektrischer Energie auszuüben, wobei die Ausübung von Druck ausreicht, um zu bewirken, dass das Arzneimittel (102) aus dem ersten Reservoir (110) ausgestoßen wird.

4. Transdermale Arzneimittelzuführungsvorrichtung gemäß Anspruch 3, **dadurch gekennzeichnet, dass** eine Diffusionsbarriere (122) an einer Grenzfläche (118) zwischen dem ersten Reservoir (110) und der mindestens einen Mikronadel (116), welche in Fluidverbindung mit dem ersten Reservoir (110) steht, positioniert ist, und dass der Zuführungsmechanismus (138) eines aus einer Einrichtung (138) zum Aufbrechen der Diffusionsbarriere (122) und einer Einrichtung (138) zum Deaktivieren der Diffusionsbarriere (122) umfasst, um **dadurch** zu ermöglichen, dass das Arzneimittel (102) aus dem ersten Reservoir (110) fließt.

5. Transdermale Arzneimittelzuführungsvorrichtung gemäß Anspruch 3, **dadurch gekennzeichnet, dass** ein Hydrogelmaterial (122) an einer Grenzfläche (118) zwischen dem ersten Reservoir (110) und der mindestens einen Mikronadel (116), die mit dem ersten Reservoir (110) in Fluidverbindung steht, positioniert ist, und der Zuführungsmechanismus (138) so konfiguriert ist, um das Hydrogelmaterial (122) schrumpfen zu lassen, um **dadurch** die Zuführung des Arzneimittels (102) von dem ersten Reservoir (110) zu ermöglichen.

6. Transdermale Arzneimittelzuführungsvorrichtung gemäß einem beliebigen der Ansprüche 3 bis 5, **dadurch gekennzeichnet, dass** der Zuführungsmechanismus (138) ein Heizelement (302) umfasst, das dafür konfiguriert ist, um eine Flüssigkeit (304) zu verdampfen, wobei die Verdampfung der Flüssigkeit (304) bewirkt, dass der Arzneistoff (102) aus dem ersten Reservoir (110) durch die mindestens eine Mikronadel (116), welche mit dem ersten Reservoir (110) in Fluidverbindung steht, ausgestoßen wird.

7. Transdermale Arzneimittelzuführungsvorrichtung gemäß einem beliebigen der Ansprüche 3 bis 5, **dadurch gekennzeichnet, dass** der Zuführungsmechanismus (138) eine Vorrichtung (310) umfasst, die dafür konfiguriert ist, um die Einleitung einer chemischen Reaktion zwischen zwei oder mehr Chemikalien (312, 314) zu ermöglichen, wobei die chemische Reaktion eine ausreichende Kraft erzeugt, um zu bewirken, dass der Arzneistoff (102) aus dem ersten Reservoir (110) ausgestoßen wird.

8. Transdermale Arzneimittelzuführungsvorrichtung gemäß einem beliebigen der vorhergehenden Ansprüche, **dadurch gekennzeichnet, dass** eine Input-Quelle (150), die so konfiguriert ist, um die Logikvorrichtung (136, 214) mit mindestens einer Zeit- und Zustandsinformation zu beliefern, bereitgestellt wird.

9. Transdermale Arzneimittelzuführungsvorrichtung gemäß Anspruch 8, **dadurch gekennzeichnet, dass** die Input-Quelle (150) mindestens eines aus einer Uhr und einem Zeitgeber umfasst und die Logikvorrichtung (136, 214) dafür konfiguriert ist, um Zeitinformationen von dem mindestens einen aus der Uhr und dem Zeitgeber zu verarbeiten und um die elektrische Energie der elektronischen Vorrichtung (138) in einer vorgeschriebenen Zeit zuzuführen.

10. Transdermale Arzneimittelzuführungsvorrichtung gemäß Anspruch 8 oder 9, **dadurch gekennzeichnet, dass** die Input-Quelle (150) einen Sensor umfasst, der dafür konfiguriert ist, um mindestens einen Zustand zu detektieren, und die Logikvorrichtung (136, 214) dafür konfiguriert ist, um die Informationen über den detektierten Zustand von dem Sensor zu verarbeiten und um die Zuführung des Arzneistoffs (102) von dem Reservoir (110) auf Basis der Informationen über den detektierten Zustand selektiv zu steuern.

11. Transdermale Arzneimittelzuführungsvorrichtung gemäß einem beliebigen der vorhergehenden Ansprüche, **dadurch gekennzeichnet, dass** die Vorrichtung weiter eine Vielzahl an ersten Reservoiren (110), wobei jedes der Vielzahl an ersten Reservoiren (110) mit einer korrespondierenden der Mikronadeln (116) in Fluidverbindung steht, und eine Vielzahl von elektronischen Vorrichtungen (138) umfasst, die mit den jeweils betreffenden aus der Vielzahl an ersten Reservoiren (110) verbunden sind, und die Logikvorrichtung (136) dafür konfiguriert ist, um elektrische Energie zu selektierten aus der Vielzahl von elektronischen Vorrichtungen (138) zuzuführen, um in den damit verbundenen ersten Reservoiren (110) der selektierten elektronischen Vorrichtungen (138) enthaltene Arzneistoffe zuzuführen.

## Revendications

1. Dispositif d'administration de médicament transdermique (100, 100') comprenant une cassette (104), la cassette (104) comprenant un premier réservoir (110) pour contenir un médicament (102) et une pluralité de micro-aiguilles (116), au moins l'une des micro-aiguilles (116) étant en communication de fluide avec le premier réservoir (110), un couvercle (106) fixé à la cassette (104) pour couvrir le premier réservoir (110), une source d'énergie (114, 144), un dispositif électronique (138) aménagé sur le couvercle (106) configuré pour recevoir l'énergie électrique générée à partir de la source d'énergie (114, 144) et un dispositif logique (136, 214) configuré pour contrôler sélectivement la distribution de l'énergie électrique au dispositif électronique (138), dans lequel la distribution de l'énergie électrique entraîne la distribution par le dispositif électronique (138) du médicament (102) contenu dans le premier réservoir (110), **caractérisé en ce que** la source d'énergie (144) comprend un matériau électrolytique (114) dans un second réservoir (112) formé dans la cassette (104) et des électrodes (146, 148) situées de telle sorte que la source d'énergie (144) devienne active lorsque le couvercle (106) est placé sur la cassette (104).

2. Dispositif de distribution de médicament transdermique selon la revendication 1, **caractérisé en ce qu'**une première (146) des électrodes est placée sur la cassette (104) et une deuxième (148) électrode est placée sur le couvercle (106), les première et deuxième électrodes (146, 148) étant placées par rapport au matériau électrolytique (114) de façon à permettre la génération d'énergie électrique lorsque le couvercle (106) est placé sur la cassette (104).

3. Dispositif de distribution de médicament transdermique selon la revendication 1 ou 2, **caractérisé en ce que** le dispositif électronique (138) comprend un mécanisme de distribution (138) configuré de façon à appliquer une pression sur le médicament (102) en réponse à la réception de l'énergie électrique, ladite application de pression étant suffisante pour entraîner l'expulsion du médicament (102) du premier réservoir (110).

4. Dispositif de distribution de médicament transdermique selon la revendication 3, **caractérisé en ce qu'**une barrière de diffusion (122) est placée à une interface (118) entre le premier réservoir (110) et au moins une micro-aiguille (116) qui est en communication de fluide avec le premier réservoir (110) et le mécanisme de distribution (138) comprend un moyen (138) permettant de rompre la barrière de diffusion (122) ou un moyen (138) permettant de désactiver la barrière de diffusion (122) pour permettre ainsi au médicament de s'écouler hors du premier réservoir (110).

5. Dispositif de distribution de médicament transdermique selon la revendication 3, **caractérisé en ce qu'**un matériau hydrogel (122) est placé sur une interface (118) entre le premier réservoir (110) et au moins l'une des micro-aiguilles (116) qui est en communication de fluide avec le premier réservoir (110) et le mécanisme de distribution (138) est configuré de façon à rétracter le matériau hydrogel (122) de façon à permettre ainsi au médicament (102) d'être distribué du premier réservoir (110).

6. Dispositif de distribution de médicament transdermique selon l'une quelconque des revendications 3 à 5, **caractérisé en ce que** le mécanisme de distribution (138) comprend un élément chauffant (302) configuré de façon à vaporiser un liquide (304), la vaporisation du liquide (304) entraînant l'expulsion du médicament (102) du premier réservoir (110) par au moins une micro-aiguille (116) qui est en communication de fluide avec le premier réservoir (110).

7. Dispositif de distribution de médicament transdermique selon l'une quelconque des revendications 3 à 5, **caractérisé en ce que** le mécanisme de distribution (138) comprend un appareil (310) configuré de façon à permettre l'initiation d'une réaction chimique entre deux ou plusieurs produits chimiques (312, 314), la réaction chimique créant une force suffisante pour entraîner l'expulsion du médicament (102) du premier réservoir (110).

8. Dispositif de distribution de médicament transdermique selon l'une quelconque des revendications précédentes, **caractérisé en ce qu'**une source de signaux d'entrée (150) configurée pour alimenter le dispositif logique (136, 214) avec au moins des informations temporelles et ou d'états est aménagée.

9. Dispositif de distribution de médicament transdermique selon la revendication 8, **caractérisé en ce que** la source de signaux d'entrée (150) comprend au moins une horloge ou une minuterie et le dispositif logique (136, 214) est configuré de façon à traiter les informations temporelles venant d'au moins l'horloge ou la minuterie et à distribuer l'énergie électrique au dispositif électronique (138) à un moment prescrit.

10. Dispositif de distribution de médicament transdermique selon la revendication 8 ou la revendication 9, **caractérisé en ce que** la source de signaux d'entrée (150) comprend un capteur configuré de façon à détecter au moins un état et le dispositif logique (136, 214) est configuré de façon à traiter l'information sur l'état détecté à partir du capteur et de contrôler sélectivement la distribution de médicament (102) depuis le réservoir (110) sur la base des informations d'état détectées.

11. Dispositif de distribution de médicament transdermique selon l'une quelconque des revendications précédentes, **caractérisé en ce que** la dispositif comprend en outre de multiples premiers réservoirs (110), chacun desdits multiples premiers réservoirs (110) étant en communication de fluide avec l'une des micro-aiguilles (116) correspondantes et une pluralité de dispositifs électroniques (138) associés à l'un desdits multiples premiers réservoirs (110) respectifs et le dispositif logique (136) est configuré de façon à distribuer une énergie électrique à l'un desdits multiples dispositifs électroniques (138) de façon à distribuer des médicaments contenus dans les premiers réservoirs associés (110) des dispositifs électroniques choisis (138).
